# EUROPÄISCHE PATENTSCHRIFT

(11) **EP 1 767 247 B1**
(45) Veröffentlichungstag und Bekanntmachung des Hinweises auf die Patenterteilung: **26.11.2008**
(21) Anmeldenummer: 05020887.5
(22) Anmeldetag: 26.09.2005
(51) Int. Cl.: A61Q 1/02, A61Q 3/02, A61K 8/34, A61K 8/81

(54) **Kosmetische Flüssigkeit zur Färbung der Nägel und der Haut**
Liquid cosmetic composition for colouring the nails and the skin
Composition cosmétique liquide pour la coloration des ongles et de la peau

(43) Veröffentlichungstag der Anmeldung: 28.03.2007
(73) Patentinhaber: Faber-Castell AG, 90546 Stein (DE)
(72) Erfinder: Appel, Tatiana, 90522 Oberasbach (DE); Lugert, Gerhard, Dr., 90431 Nürnberg (DE); Schwarz, Wolfgang, 90522 Ansbach (DE)
(74) Vertreter: Mörtel & Höfner

(56) Entgegenhaltungen:
- WO-A-20/04071510
- DE-A1- 3 810 897
- US-A1- 2005 063 909

## Beschreibung

Die Erfindung betrifft eine kosmetische Flüssigkeit zur Färbung der Haut, der Finger- und der Fußnägel, d.h. einen Nagellack und eine kosmetische Färbeflüssigkeit, wobei mit letzterer beispielsweise Tattoo ähnliche Zeichnungen erzeugbar sind. Herkömmliche Nagellacke enthalten allgemein einen Filmbildner, beispielsweise Ketonformaldehydharze, Epoxyharze oder Nitrocellulose, der in einem schnell verdampfenden Lösungsmittel, beispielsweise Ester, Ketone, manchmal auch in Mischung mit Alkoholen gelöst oder emulgiert ist. Eine Applikation erfolgt in der Regel durch Eintauchen von Pinseln oder Schwämmchen in die Lacke, welche in Glas- oder Kunststofffläschchen aufbewahrt werden. Nach dem Auftragen mit Hilfe des Pinsels verdampft das Lösungsmittel und es bleibt ein fester Film zurück. Um eine im Regelfall gewünschte Deckwirkung zu erzielen muss eine relativ große Nagellackmenge aufgebracht werden. Dies gelingt in zufriedenstellender Weise nur, wenn der Nagellack relativ dickflüssig ist, d.h. wenn er eine Viskosität von mehr als 100 mPas (Brookfield; 25°C) aufweist.

Nachteilig bei den bekannten Lacken und dem üblichen Applikationssystem ist, dass die Applikation einiges Geschick erfordert, um ein Verschütten des Fläschcheninhalts zu vermeiden. Ein weiterer Nachteil ist, dass mit einem Pinsel oder einem Schwämmchen oft eine zu große Lackmenge aufgenommen und der Lacküberschuss am Öffnungsrand des Fläschchens abgestreift wird. Der Öffnungsbereich des Fläschchens wird dabei mit fortschreitender Gebrauchsdauer durch eintrocknenden Lack verschmutz und verengt. Außerdem besteht die Gefahr, dass der Lack außen am Fläschchen herunter läuft und das im Öffnungsbereich vorhandene Gewinde zum Aufschrauben der Verschlusskappe verschmutzt. Bei Nagellacken mit Farbpigmenten ist problematisch, dass sich die anfänglich homogen im Lack verteilten Pigmente abtrennen und der Nagellack inhomogen wird. Nachteilig ist weiterhin, dass während der Applikation das Fläschchen offen ist und Lösungsmittel verdampfen kann. Mit der Zeit wird dadurch der Nagellack immer zähflüssiger. Die relativ hohe Viskosität in Verbindung mit der zur Erzeugung eines deckenden Films erforderlichen großen Lackmenge haben unerwünscht lange Trockenzeiten zur Folge.

Davon ausgehend ist es die Aufgabe der Erfindung, eine kosmetische Flüssigkeit vorzuschlagen, die bei verbesserter Applikation als Nagellack und darüber hinaus zur Färbung der Haut geeignet ist.

Diese Aufgabe wird nach Anspruch 1 mit einer Flüssigkeit gelöst, die als filmbildendes Polymer wenigstens ein Acrylat/Dimethylaminoethyl-Methylacrylat-Copolymer mit einem Anteil von 5 % bis 15 % und ein aus wenigstens einem Alkohol gebildetes Lösungsmittel mit einem Anteil von 65 % bis 85 % enthält, und die eine Viskosität von weniger als 100 mPas, vorzugsweise weniger als 50 mPas (Brookfield; 25°C) aufweist.

Überraschenderweise hat sich gezeigt, dass die Verwendung von Polymethylacrylaten der beanspruchten Art in Verbindung mit Alkohol oder in wässrigen Alkohol-Lösungen als Lösungsmittel keinen signifikanten Viskositätsaufbau bewirken, dass sie aber dennoch Filme mit einer für eine farbliche Überdeckung des Untergrunds erforderlichen Dicke und Konsistenz ausbilden.

Gegenüber herkömmlichen Nagellacken ist zur Erzeugung eines Films eine vergleichsweise geringe Menge an Flüssigkeit erforderlich, was in Verbindung mit der geringen Viskosität ein schnelleres Verdampfen des Lösungsmittels bei entsprechend verringerten Trockenzeiten erlaubt.

Alle diese Eigenschaften prädestinieren die vorgeschlagene Flüssigkeit für eine Applikation mit Hilfe eines kapillaren Auftragsgeräts. Unter einem Kapillarsystem sind kapillare Faserspeicher aus Kunststofffasern, etwa aus Polyester oder Polypropylen zu verstehen, die in Metall- oder Kunststoffschäften eingesetzt werden. Eine in die Speicher eintauchende Faser oder Sinterspitze transportiert die innerhalb der Speicher vorhandenen Lacke bzw. Flüssigkeiten auf Fuß- oder Fingernägel oder auf die Haut. Insbesondere im Falle von Nagellacken erleichtert die Erfindung die Applikation erheblich. So entfällt die problembehaftete Handhabung des Fläschchen, die Flüssigkeit kann schnell und bedingt durch die eine exakte Geometrie aufweisende Faser- oder Sinterspitze sehr exakt, tropffrei und gleichmäßig aufgetragen werden. Eine Applikation herkömmlicher Nagellacke mit kapillaren Auftragsgeräten war bisher aufgrund der hohen Viskosität der Lacke nicht möglich. Die Verwendung dünnflüssiger, also mit Lösungsmitteln verdünnter Lack hilft nicht weiter, weil sich damit keine deckenden Aufträge, zumindest nicht bei einmaligem Auftrag erzeugen lassen. Außerdem würde die Kapillarspitze eines Auftragsgerätes aufgrund der hohen Volatilität der Lösungsmittel mit getrocknetem Lack schnell verstopfen.

Die mit einer erfindungsgemäßen Flüssigkeit erzeugten Filme sind homogen, weisen einen für Nagellackierungen gewünschten Glanz auf, sind permanent und wasserfest. Sie lassen sich durch Alkohole oder Alkohol-Wasser-Lösungen leicht wieder entfernen, wobei eine übermäßige Entfettung der Haut und eine Versprödung der Nägel, wie bei den mit Lösungsmitteln wie Ketonen oder Ester arbeitenden Nagellacken, nicht auftritt. Die vorgeschlagenen Flüssigkeiten sind aufgrund ihrer toxikologisch unbedenklichen Lösungsmittel auch in gesundheitlicher Hinsicht verbessert. Als umweltverträgliche und toxikologisch unbedenkliche Alternativen sind zwar Nagellacke auf Wasserbasis bekannt, diese bilden jedoch einen zu geringen Glanz aus, besitzen eine zu geringe Permanenz und erfordern lange Trockenzeiten.

Vorteilhaft ist weiterhin, dass im Falle einer Einfärbung mit Farbpigmenten diese in der Flüssigkeit auch bei längerer Lagerung in homogener verteilung gehalten werden, eine sedimentierung wie bei den oben beschriebenen Nagellacken also nicht auftritt. Darüber hinaus ist es aufgrund der hohen Alkoholgehalte nicht notwendig zusätzliche Konservierungsmittel innerhalb der Formulierung zu verwenden.

Überraschenderweise hat sich schließlich gezeigt, dass bei Verwendung von Polymethylacrylaten der beanspruchten Art in Alkoholen zusammen mit Farbmitteln Hautmarkierflüssigkeiten hergestellt werden können, die mit großer Exaktheit applizierbar sind und einen sehr schönen, zum Teil sogar metallischen Glanz auf der Haut ausbilden können.

Auf der Haut aufgebrachte Einfärbungen beispielsweise Tattoos, sind gut spritzwasserfest und längere Zeit auf der Haut sichtbar.

Die Flüssigkeiten enthalten einen Anteil an Acrylat/Dimethylaminoethyl-Methylacrylat-Copolymer von 5 % bis 15 % und einem Alkoholgehalt von 65 % bis 85 %. Solche Flüssigkeiten eignen sich in besonderer Weise für eine Applikation mit einem kapillaren Auftragsgerät.

Wie bereits erwähnt, kann gegebenenfalls ein mit Wasser verdünnter Alkohol eingesetzt werden, wobei im Hinblick auf möglichst kurze Trockenzeiten eine Obergrenze des Wassergehaltes von 20 % eingehalten werden sollte. Als Alkohole werden bevorzugt Ethanol, Propanol und 1-Methoxy-2-Propanol verwendet.

Durch Zusatz eines weiteren, etwa von herkömmlichen Nagellacken bekannten Filmbildners lassen sich, falls erforderlich, die Eigenschaften von Filmen etwa deren Härte, Dauerhaftigkeit, Haftung und Glanz verändern. Vorzugsweise werden Harze aus der Gruppe Toluensulfonamid-Epoxyharze, Polyesterharze und Ketonformaldehydharze mit einem Anteil von max. 25 % in der Flüssigkeit gelöst.

Der Anteil von Farbstoffen oder Farbpigmenten ergibt sich aus deren jeweiligen Färbekraft und den gewünschten Farbsättigungen. Vorzugsweise wird deren Anteil aber auf max. 10 % begrenzt.

Als Additive sind Aromen, Parfums aber auch Dispegierhilfsmittel. denkbar, wobei vorzugsweise ein Maximalgehalt von 10 % eingehalten wird. Zur Verbesserung der Fließeigenschaften und der Applikation ist ein Zusatz von polyethermodifizierten Polysiloxanen als Additiv mit einem Anteil von 0,5 % bis 3 %, insbesondere von PPG/PEG 4/12 Dimethicon (INCI-Bezeichnung), besonders empfehlenswert.

Eine erfindungsgemäße Flüssigkeit lässt sich zusammenfassend durch die folgende Basisrezeptur wiedergeben.

| | |
|---|---|
| Acrylat/Dimethylaminoethyl-Methylacrylat Copolymer | 5 - 15 % |
| Zusätzlicher Filmbildner | 0 - 25 % |
| Alkohole | 65 - 85 % |
| Wasser | 0 - 20 % |
| Additive | 0 - 10 % |
| Farbmittel | 0 - 10 % |

### Beispiel 1:

Farbloser Lack zur Applikation auf Fuß- oder Fingernägeln

| | |
|---|---|
| Ethanol | 64,0 % |
| 1-Methoxy-2-Propanol | 20,0 % |
| Acrylat/Dimethylaminoethyl Methylacrylat Copolymer ¹⁾ | 10,0 % |
| Toluensulfonamid-Epoxyharz ²⁾ | 5,0 % |
| PPG/PEG 4/12 Dimethicone ³⁾ | 1,0 % |

zur Herstellung wird das Polyacrylat-Copolymer und das Toluensulfonamid-Epoxyharz unter leichter Erwärmung und Rühren in dem Alkohol gelöst. Anschließend erfolgt der Zusatz des Additivs PPG/PEG 4/12 Dimethicone.

### Beispiel 2:

Transparent eingefärbte rote Nagellasur

| | |
|---|---|
| Ethanol | 63,8 % |
| 1-Methoxy-2-Propanol | 20,0 % |
| Acrylat/Dimethylaminoethyl Methylacrylat Copolymer ¹⁾ | 10,0 % |
| Toluensulfonamid Epoxy Harz ²⁾ | 5,0 % |
| PPG/PEG 4/12 Dimethicone ³⁾ | 1,0 % |
| C.I. Acid Red 87 C.I. No. 45380 | 0,1 % |
| C.I. Acid Red 92 C.I. No. 45410 | 0,1 % |

### Beispiel 3:

Orange eingefärbte Nagellasur. Sie bildet wasserfeste transparente Filme aus.

| | |
|---|---|
| Ethanol | 64,7 % |
| 1-Propanol | 20,0 % |
| Acrylat/Dimethylaminoethyl Methylacrylat Copolymer ⁴⁾ | 15,0 % |
| C.I. Acid Red 92, C.I. No 45410 | 0,1 % |
| C.I. Food Yellow 3, C.I. No 15985 | 0,1 % |
| Aroma | 0,1 % |

Die Herstellung hier und bei Beispiel 2 erfolgt entsprechend Beispiel 1.

### Beispiel 4:

Roter Nagellack

| | |
|---|---|
| Ethanol | 70,9 % |
| Keton-formaldehyd-Harz ⁵⁾ | 20,0 % |
| Acrylat/Dimethylaminoethyl Methylacrylat Copolymer ¹⁾ | 5,0 % |
| PPG/PEG 4/12 Dimethicone ³⁾ | 0,5 % |
| Parfum | 0,1 % |
| C.I. 15850:1 | 3,5 % |

Das Pigment C.I. 15850:1 (D&C Red7 Calciumlake) wird mit einer Kugelmühle gemahlen, um die Pigmentpartikel zwecks einer Applikation mit einem kapillaren Auftragsgerät zu zerkleinern. Ansonsten erfolgt die Herstellung wie weiter oben beschrieben.

### Beispiel 5:

Blaue Tattoo-Flüssigkeit mit metallischem Glanz

| | |
|---|---|
| Ethanol | 69,0 % |
| Wasser | 15,0 % |
| Acrylat/Dimethylaminoethyl-Methylacrylat-Copolymer ¹⁾ | 15,0 % |
| Acid Blue 9 C.I. No. 42090 | 1,0 % |

Herstellung erfolgt wie bei Beispiel 1.
1) Eudragit E100; Hersteller: Röhm GmbH, D-64275 Darmstadt
2) Polytex E 100; Hersteller: Worlée Chemie GmbH, D-22113 Hamburg
3) ABIL B 8852; Hersteller: Goldschmidt / Degussa, Essen
4) Jurymer ET-410 D, Hersteller: Nihon Junyaku, Tokyo/Japan
5) Kunstharz SK, Hersteller: Degussa AG

Alle Prozentangaben sind Gewichtsprozent.

## Patentansprüche

1. Kosmetische Flüssigkeit zur Färbung der Haut, der Finger- und der Fußnägel mit einem Lösungsmittel und einem darin gelösten filmbildenden Polymer,
**dadurch gekennzeichnet,**
**dass** als Filmbildner wenigstens ein Acrylat/Dimethylaminoethyl-Methylacrylat-Copolymer mit einem Anteil von 5 % bis 15 % und ein aus wenigstens einem Alkohol gebildetes Lösungsmittel mit einem Anteil von 65 % bis 85 % enthalten sind, wobei die Flüssigkeit eine Viskosität von weniger als 100 mPas (Brookfield; 25°C) aufweist.

2. Kosmetische Flüssigkeit nach Anspruch 1,
**dadurch gekennzeichnet,**
**dass** sie bis zu 20 % Wasser enthält.

3. Kosmetische Flüssigkeit nach einem der vorhergehenden Ansprüche,
**dadurch gekennzeichnet,**
**dass** als zweiter Filmbildner wenigstens ein Harz aus der Gruppe Toluensulfonamid-Epoxyharze, Polyesterharze, Ketonformaldehydharze mit einem Anteil von max. 25 % in gelöster Form enthalten ist.

4. Kosmetische Flüssigkeit nach einem der vorhergehenden Ansprüche,
**dadurch gekennzeichnet,**
**dass** ein Farbpigment mit einem Anteil von max. 10 % enthalten ist.

5. Kosmetische Flüssigkeit nach Anspruch 4,
**dadurch gekennzeichnet,**
**dass** ein in Alkohol oder einem Alkohol-Wasser-Gemisch löslicher Farbstoff mit einem Anteil von max. 10 % enthalten ist.

6. Kosmetische Flüssigkeit nach einem der vorhergehenden Ansprüche,
**dadurch gekennzeichnet,**
**dass** als Lösungsmittel ein oder mehrere Alkohole aus der Gruppe Ethanol, Propanol und 1-Methoxy-2-Propanol enthalten ist.

7. Kosmetische Flüssigkeit nach einem der vorhergehenden Ansprüche,
**dadurch gekennzeichnet,**
**dass** wenigstens ein Additiv mit einem Anteil von max. 10 % enthalten ist.

8. Kosmetische Flüssigkeit nach Anspruch 7,
**dadurch gekennzeichnet,**
**dass** ein polyethermodifiziertes Polysiloxan mit einem Anteil von 0,5 bis 3 % enthalten ist.

9. Kosmetische Flüssigkeit nach Anspruch 8,
**dadurch gekennzeichnet,**
**dass** PPG/PEG 4/12 Dimethicon enthalten ist.

10. Kosmetische Flüssigkeit nach einem der vorhergehenden Ansprüche,
**dadurch gekennzeichnet,**
**dass** sie eine Viskosität von weniger als 50 mPas (Brookfield; 25°C) aufweist.

## Claims

1. Cosmetic liquid for colouring the skin, the fingernails and the toenails having a solvent and a film-forming polymer dissolved therein,
**characterized in that**
at least one acrylate/dimethylaminoethyl methylacrylate copolymer with a fraction of from 5% to 15% and a solvent formed from at least one alcohol with a fraction of from 65% to 85% are present as film former, where the liquid has a viscosity of less than 100 mPas (Brookfield; 25°C) .

2. Cosmetic liquid according to Claim 1,
**characterized in that**
it comprises up to 20% water.

3. Cosmetic liquid according to one of the preceding claims,
**characterized in that**
at least one resin from the group toluene-sulphonamide epoxy resins, polyester resins, ketone formaldehyde resins with a fraction of at most 25% in dissolved form is present as a second film former.

4. Cosmetic liquid according to one of the preceding claims,
**characterized in that**
a coloured pigment with a fraction of at most 10% is present.

5. Cosmetic liquid according to Claim 4,
**characterized in that**
a dye which is soluble in alcohol or an alcohol/water mixture with a fraction of at most 10% is present.

6. Cosmetic liquid according to one of the preceding claims,
**characterized in that**
one or more alcohols from the group ethanol, propanol and 1-methoxy-2-propanol is present as solvent.

7. Cosmetic liquid according to one of the preceding claims,
**characterized in that**
at least one additive with a fraction of at most 10% is present.

8. Cosmetic liquid according to Claim 7,
**characterized in that**
a polyether-modified polysiloxane with a fraction of from 0.5 to 3% is present.

9. Cosmetic liquid according to Claim 8,
**characterized in that**
PPG/PEG 4/12 dimethicone is present.

10. Cosmetic liquid according to one of the preceding claims,
**characterized in that**
it has a viscosity of less than 50 mPas (Brookfield; 25°C).

## Revendications

1. Liquide cosmétique de coloration de la peau, de l'ongle du doigt et de l'ongle du pied, comprenant un solvant et un polymère filmogène qui y est dissous,
**caractérisé**
**en ce qu'**il est contenu comme agent filmogène au moins un copolymère d'acrylate/méthacrylate de diméthylaminoéthyle en une proportion de 5 % à 15 % et un solvant formé d'au moins un alcool en une proportion de 65 % à 85 %, le liquide ayant une viscosité de moins de 100 mPas ( Brookfield ; 25°C ).

2. Liquide cosmétique suivant la revendication 1,
**caractérisé**
**en ce qu'**il contient jusqu'à 20 % d'eau.

3. Liquide cosmétique suivant l'une des revendications précédentes,
**caractérisé**
**en ce qu'**il est contenu comme deuxième agent filmogène au moins une résine du groupe des résines toluènesulfonamide-époxy, des résines de polyester, des résines cétoneformaldéhyde en une proportion de 25 % au maximum sous forme dissoute.

4. Liquide cosmétique suivant l'une des revendications précédentes,
**caractérisé**
**en ce qu'**il est contenu un pigment coloré en une proportion de 10 % au maximum.

5. Liquide cosmétique suivant la revendication 4,
**caractérisé**
**en ce qu'**il est contenu un colorant soluble dans un alcool ou dans un mélange d'alcool et d'eau en une proportion de 10 % au maximum.

6. Liquide cosmétique suivant l'une des revendications précédentes,
**caractérisé**
**en ce qu'**il est contenu comme solvant un alcool ou plusieurs alcools du groupe de l'éthanol, du propanol et du 1-méthoxy-2-propanol.

7. Liquide cosmétique suivant l'une des revendications précédentes,
**caractérisé**
**en ce qu'**il est contenu au moins un additif en une proportion de 10 % au maximum.

8. Liquide cosmétique suivant la revendication 7,
**caractérisé**
**en ce qu'**il est contenu un polysiloxane modifié par un polyéther en une proportion de 0,5 à 3 %.

9. Liquide cosmétique suivant la revendication 8,
**caractérisé**
**en ce qu'**il est contenu du PPG/PEG 4/12 diméthicone.

10. Liquide cosmétique suivant l'une des revendications précédentes,
**caractérisé**
**en ce qu'**il a une viscosité de moins de 50 mPas ( Brookfield ; 25°C ).
